Europäisches Patentamt

European Patent Office

Office européen des brevets

(19)

(11) Numéro de publication: **0 518 765 B1**

(12) **FASCICULE DE BREVET EUROPEEN**

(45) Date de publication de fascicule du brevet: **28.12.94**

(51) Int. Cl.5: **C07C 43/13**, C07C 41/09, C11B 3/00

(21) Numéro de dépôt: **92401615.7**

(22) Date de dépôt: **10.06.92**

(54) **Procédé pour l'obtention de polyglycérols et d'esters de polyglycérols.**

(30) Priorité: **12.06.91 FR 9107175**

(43) Date de publication de la demande:
**16.12.92 Bulletin 92/51**

(45) Mention de la délivrance du brevet:
**28.12.94 Bulletin 94/52**

(84) Etats contractants désignés:
**AT BE DE DK ES FR GB GR IT NL PT SE**

(56) Documents cités:
**EP-A- 0 151 755**
**US-A- 3 637 774**

(73) Titulaire: **ORGANISATION NATIONALE INTER-PROFESSIONNELLE DES OLEAGINEUX- ONI-DOL**
**12, avenue George V**
**F-75008 Paris (FR)**

(72) Inventeur: **Hillion, Gérard**
**10, Place du Cassan**
**F-95220 Herblay (FR)**
Inventeur: **Stern, Robert**
**6bis, rue des Côtes de Vannes**
**F-78700 Conflans Ste-Honorine (FR)**

(74) Mandataire: **Phélip, Bruno**
**c/o Cabinet Harlé & Phélip**
**21, rue de La Rochefoucauld**
**F-75009 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention a pour objet un procédé pour l'obtention de polyglycérols ainsi que d'esters de polyglycérols .

Il est connu de fabriquer des polyglycérols , qui sont des éthers de glycérine , par chauffage de glycérine pure en présence de catalyseurs acides ou basiques . De même , on sait préparer des esters de polyglycérols par action d'acides gras , d'esters ou d'huiles sur des polyglycérols . Ces procédés sont connus depuis longtemps . A titre illustratif , des documents représentatifs de la technique antérieure sont par exemple les brevets US 2.284.127 et 3 637.774.

Les polyglycérols sont des liquides assez visqueux qui ont la propriété de retenir l'eau et peuvent servir d'humectants , d'agents antistatiques, d'antimottants, de solvants mais surtout de matière première pour fabriquer des esters qui possèdent des propriétés amphiphiles.

Ceux-ci sont utilisés dans de nombreuses industries où le pouvoir émulsifiant , antimoussant , ou lubrifiant est important : dans la boulangerie, la pâtisserie, les matières plastiques , les colles etc.

Ainsi , les polyglycérols et leurs esters sont connus de l'homme du métier , de même que leurs principales applications . On peut se référer à cet égard à l'article de Behrens et Mieth ( Die Nahrung, 28 , (8), 815-835, 1984 ) .

Ces produits , souvent biodégradables , souffrent cependant d'un handicap sérieux : le prix de la fabrication des polyglycérols qui dépend , selon certaines données de la littérature , pour plus de 70% du coût de la glycérine pure .

La glycérine brute du commerce , moins coûteuse , est malheureusement inutilisable car elle contient plusieurs pour cent de sels d'acides forts comme le sulfate et le chlorure de sodium . A haute température c'est-à-dire entre 220 et 270°C température où les polyglycérols se forment , ces glycérines se décomposent en donnant entre autres et abondamment de l'acroléine. De plus , les produits obtenus sont très sombres .

L'invention a pour objet un nouveau procédé de fabrication de polyglycérols et d'esters de polyglycérols qui permet d'obtenir ces produits de manière beaucoup moins coûteuse que dans l'art antérieur . Le procédé de l'invention fournit de manière directe et rapide des produits qui , de manière surprenante , ont une coloration plus claire que celle des produits analogues fabriqués par les procédés connus .

D'une manière générale , l'invention a pour objet un procédé pour l'obtention de polyglycérols et d'esters de polyglycérols comprenant l'étape de chauffage d'une matière première glycérineuse brute , provenant d'une réaction d'estérification , en soi connue , d'une huile et d'un monoalcool en présence d'un catalyseur basique.

L'enseignement de l'invention consiste donc essentiellement à utiliser la phase glycérineuse obtenue lors de la fabrication d'esters des corps gras à partir d'huiles végétales ou animales , en présence d'un catalyseur basique .

Plus précisément la phase utilisée est celle qui décante lorsqu'on soumet une huile à une transestérification avec un monoalcool, tel que le méthanol , l'éthanol , le propanol ou le butanol en présence de soude , de potasse ou d'alcoolates alcalins.

L'huile qui a servi à la production de la phase glycérineuse est une huile végétale ou animale quelconque susceptible de subir une estérification ou une transestérification sous l'action d'un monoalcool en présence d'un catalyseur basique . Il peut s'agir d'une huile végétale ou animale , telle que les huiles de palmiste, coprah , babassu , palme , suif , saindoux , tournesol , colza , lin, arachide , coton, carthame , poisson, soja, tall oil , seules ou en mélange .

A titre de monoalcools , on utilise de préférence le méthanol ou l'éthanol . Les autres alcools inférieurs , tels que le propanol ou le butanol, bien qu'également utilisables, sont moins avantageux

Les phases glycérineuses obtenues après la ou les réactions de transestérification présentent une concentration assez élevée en ions alcalins , qui est due au fait que l'on introduit dans l'alcool et l'huile , pour la transestérification , un catalyseur qui , selon l'huile , est présent à une concentration molaire de 40 à 400 millimoles d'ions alcalins par kg d'huile . Pendant la réaction, le catalyseur passe presque complètement dans la phase glycérineuse , ce qui explique qu'il se concentre dans cette phase . Lorsque la glycérine constitue avec certaines huiles , 10% du poids de l'huile , le catalyseur se trouve à une concentration fortement augmentée par rapport à la glycérine . Toutefois , il n'est généralement plus sous la forme de base libre ni sous la forme d'alcoolate mais plutôt sous la forme d'un sel alcalin d'acide gras . Les acides gras proviennent de la réaction avec l'huile , qui a servi à fabriquer les esters.

A titre illustratif , la phase glycérineuse utilisée comme matière première dans le procédé de l'invention contient par exemple 50% de glycérine , 20% de sels d'acides gras, d'esters entraînés ou de monoglycérides et 30% de méthanol et d'eau , si l'on a ajouté de l'eau au cours des traitements . La concentration en ions sodium ou potassium par rapport à la glycérine est de l'ordre de 0,4 à 4 moles d'ions alcalins par kg de glycérine ( ou la moitié si l'on considère la phase glycérineuse donnée comme exemple) .

L'invention tire profit de la composition d'une telle phase glycérineuse brute , le procédé proposé procurant des avantages nombreux et importants . L'avantage le plus décisif est de caractère économique . Alors que , dans la technique antérieure , seule de la glycérine pure et coûteuse pouvait être utilisée , l'invention permet de traiter directement une glycérine brute . En outre , au cours de la formation des polyglycérols , les sous-produits s'éliminent d'eux mêmes et peuvent être récupérés . De telles caractéristiques de l'invention sont particulièrement intéressantes dans le cas où la glycérine est disponible sous forme d'un sous-produit de la production de certains carburants. La purification , qui implique parfois une distillation multiple , de la glycérine , entraîne un coût prohibitif pour valoriser un tel produit . Grâce au procédé de l'invention, il devient possible de traiter directement la phase glycérineuse , en obtenant des polyglycérols d'excellente qualité . En effet, on a constaté de manière très surprenante que la coloration de tels polyglycérols est plus claire que celle obtenue à partir de glycérine distillée et d'un réactif alcalin telle que la soude ( hydroxyde de sodium ou carbonate de sodium ) jouant le rôle de catalyseur basique. Au fur et à mesure que la réaction du procédé de l'invention se déroule, la couleur du polyglycérol s'éclaircit progressivement et le produit final a une coloration nettement moins sombre que celle des produits analogues connus . Sans que cette théorie puisse constituer une limitation quelconque à la portée de l'invention , on pense que cet avantage peut être attribué au fait que la réaction de formation des polyglycérols s'effectue dans des conditions de catalyse douce. Il n'est en effet pas nécessaire d'ajouter un catalyseur à la matière première glycérineuse , celle-ci contenant , comme on l'a indiqué ci-dessus , une concentration suffisamment élevée en ions alcalins , qui sont présents sous forme de savons provenant des acides gras.

Les produits obtenus par le procédé de l'invention sont des mélanges de polyglycérols dont la composition varie selon l'huile de départ .

Selon l'invention, le procédé utilisé pour fabriquer un polyglycérol à partir de la phase glycérineuse précitée , peut comprendre plusieurs modes de réalisation.

Dans un premier mode de réalisation , on prend les phases glycérineuses provenant de la transestérification des huiles et qui n'ont subi aucun traitement neutralisant avec un acide fort . On chauffe ces phases jusqu'à 200°C environ en évaporant le monoalcool, tel que le méthanol , et l'eau présente , puis on continue à chauffer jusqu'à 250-270°C en recueillant l'eau de condensation. L'acide gras présent sous forme de sel ne réagit pas , mais le sel catalyse la réaction de polycondensation de la glycérine . Pour éliminer le catalyseur , on ajoute à basse température un acide fort , qui libère l'acide gras que l'on décante. Si l'on utilise de l'acide sulfurique , on peut ajouter de l'alcool et filtrer le sulfate de sodium . Les traces sont éliminées par passage sur résines . Le polyglycérol obtenu peut être analysé par silylation et CPV . Il comprend un mélange de polyglycérols dont le poids moléculaire dépend de la quantité d'eau éliminée dans la condensation. Il s'agit généralement de polyglycérols de 2 à 10 chaînons de glycérine . Le polyglycérol est jaune clair alors que la glycérine au départ était orange foncé.

Dans un autre mode de réalisation, on traite la phase glycérineuse par un acide carboxylique faible , dont le pK est inférieur à celui d'un acide gras , tel que l'acide acétique , en stoéchiométrie par rapport aux ions alcalins . On décante l'acide gras libéré après ou avant évaporation de l'alcool de type monoalcool généralement présent , et l'on continue à chauffer jusqu'à 250-260°C en éliminant l'eau que l'on compatibilise à partir de 200-220°C . On élimine de la même manière l'acétate de sodium par déplacement du sel à basse température avec un acide fort en présence d'un alcool et on traite comme dans le premier mode de réalisation.

Si l'on désire fabriquer un ester de polyglycérol, on peut également opérer suivant plusieurs variantes .

Dans le premier type de procédé on peut , après formation du polyglycérol , refroidir à 200°C et injecter un acide gras ou un ester quelconque , laisser réagir , refroidir, neutraliser, filtrer le catalyseur en présence d'un solvant apolaire puis comme précédemment passer sur des colonnes de résines échangeuses d'ions .

L'inconvénient de ce procédé est qu'il libère au refroidissement après neutralisation un acide gras ou de l'acide acétique . S'il s'agit de l'acide acétique , il est possible de l'éliminer sans problème mais s'il s'agit d'acide gras , il sera présent dans les esters de polyglycérols.

Dans un deuxième procédé , après formation de polyglycérol selon le premier mode de réalisation, on refroidit à 200°C et on ajoute l'acide acétique à ce stade . Il se libère les acides gras correspondant à la stoéchiométrie introduite . Ces acides gras réagissent à cette température avec le polyglycérol . On rajoute ensuite l'ester ou l'acide gras que l'on veut lier au polyglycérol . On peut aussi inverser l'introduction et introduire l'acide acétique après introduction de l'acide ou de l'ester de corps gras . La réaction est assez rapide et l'acide ou l'ester réagissent dans des temps courts de 15 minutes à 1 heure à cette température .

3

On refroidit ensuite et l'on élimine les ions alcalins avec un acide minéral en milieu hydrocarbure . L'inconvénient de ce procédé est que l'on risque d'avoir plusieurs types d'esters de polyglycérols, si la nature de l'acide gras est différente, c'est-à-dire si les acides gras du sel ne sont pas identiques à ceux introduits lors de l'estérification. Une variante qui permet de pallier cette difficulté consiste à introduire l'acide acétique à 150°C environ et à décanter l'acide libre libéré avant d'introduire à 200°C un ester ou un acide gras .

Enfin une dernière variante consiste à fabriquer un polyglycérol et à le purifier complètement . On peut en outre distiller la glycérine et les dérivés cycliques présents , de manière à obtenir un polyglycérol qui se comporte mieux dans les propriétés tensioactives puisqu'il contient peu de glycérine .

Toutefois , dans ce cas , il est préférable de rajouter un catalyseur tel que la soude pour accélérer la formation d'esters de polyglycérol , le catalyseur ayant été éliminé dans la purification.

Le procédé de fabrication de polyglycérol selon l'invention peut se prêter à une conduite en continu dans un dispositif qui permet d'évaporer l'eau en continu , par exemple dans une colonne à film tombant , et de recycler le produit .

L'invention sera encore illustrée , sans être aucunement limitée , par les exemples suivants dans lesquels :

La figure 1 représente le schéma de fabrication d'un pentaglycérol selon l'exemple 1 ou 4.

La figure 2 représente le schéma de fabrication d'un polyglycérol après neutralisation par l'acide acétique selon l'exemple 2 .

La figure 3 est un diagramme illustrant la fabrication à titre comparatif de pentaglycérol à partir de glycérine bidistillée et d'hydroxyde de sodium selon l'exemple 3 .

La figure 4 représente le schéma de fabrication d'un ester mixte de coprah et de colza de pentaglycérol selon l'exemple 5 .

La figure 5 représente le schéma de fabrication d'un ester de coprah de polyglycérol selon l'exemple 6.

EXEMPLE 1 :

Fabrication d'un pentaglycérol.

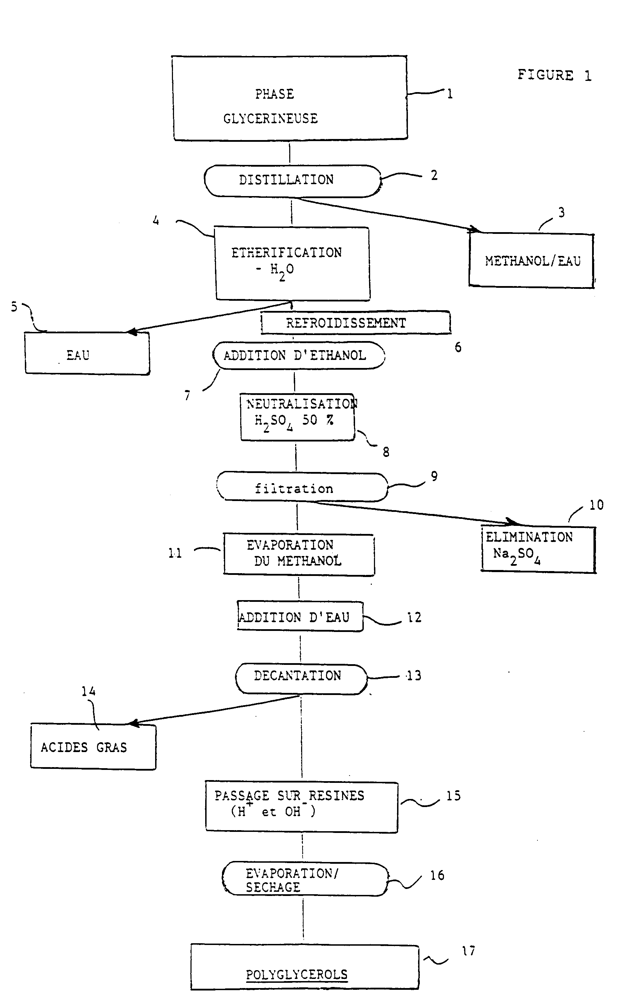

Comme indiqué sur la figure 1 , 700 g de phase glycérineuse (1) comprenant 52,4 mmol d'ions sodium pour 100 g de solution et 50,4 % de glycérine en poids soit 352,7 g , sont chauffés dans un ballon surmonté d'une colonne à reflux et d'un condenseur . Un léger courant d'argon est introduit dans le ballon qui, de plus, est muni d'un système qui permet une agitation suffisante . La distillation ( 2 ) jusqu'à 235°C donne 278,4 g d'un mélange eau + alcool ( 3 ) . On continue à chauffer entre 230 et 255°C ( 4 ) et l'on recueille en l'espace de 83 minutes 55 ml d'eau ( 5 ) , ce qui correspond à la stoéchiométrie , pour obtenir un pentaglycérol . On refroidit ( 6 ) et à 150°C on introduit de l'alcool éthylique ( 7 ) et à 60°C une solution à 50% d'acide sulfurique correspondant à l'alcalinité de départ ( 8 ) .Après filtration ( 9) élimination de $Na_2SO_4$ (10 ) évaporation de l'alcool (11) et reprise par de l'eau (12 ) ( 550 ml ) on décante une phase supérieure (13 ) ( 69,5 g ) qui contient 71% d'acide gras soit 44,2 g d'acide olé'ique ( 14 ) . La phase aqueuse ( 891 g) est passée sur résines échangeuses d'ions ( 15 ) . Après évaporation ( 16 ) , on obtient 242 g d'un polyglycérol jaune pâle et légèrement trouble à froid ( 17 ) . Le trouble provient vraisemblablement de traces d'esters de polyglycérols ( couleur Lovibond = 2,5) . En bilan poids on a obtenu 81,4% de rendement . Les pertes proviennent des produits qui restent sur le précipité et dans les colonnes. Le polyglycérol après silylation est passé sur une colonne de chromatographie en phase vapeur (CPV) qui montre la présence de très nombreux isomères de polyglycérols de poids moléculaire très variés (voir tableau I ci-après ).

EXEMPLE 2 :

Fabrication d'un polyglycérol après neutralisation à l'acide acétique .

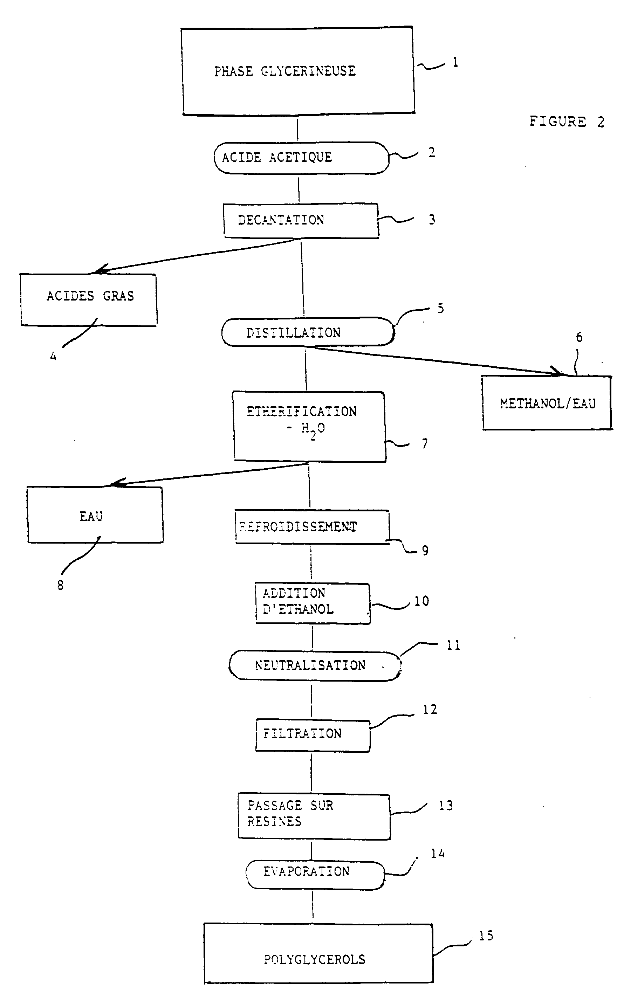

Comme indiqué sur le diagramme de la figure 2 , on neutralise à 30°C à l'acide acétique ( 2 ) (25,5g) la phase glycérineuse ( 1 ) (750g) provenant des deux premières transestérifications d'une huile de colza en ester méthylique. Il se forme deux phases ( 3 ) , la phase surnageante comprend les acides gras ( 4 ) et l'ester entraîné dans la glycérine soit près de 20% de cette phase . On évapore les 600 g de la phase inférieure qui comprend presque tout l'acétate de sodium formé dans la réaction. On chauffe jusqu'à 230°C ( 5 ) et l'on recueille 207 g du mélange eau - alcool ( 6 ) . Il reste 362 g de glycérine que l'on chauffe encore jusqu'à 255° ( 7 ) . On recueille 56,6 g d'eau en 4 h 50 ( 8 ) . On refroidit ( 9 ) , on ajoute à 150°C,

EP 0 518 765 B1

200g d'éthanol ( 10 ) , et à froid 41g d'acide sulfurique à 50% ( 11 ) . Après filtration du sulfate de sodium ( 12 ) passage sur résines (13 ) , évaporation de l'alcool (14 ) , on obtient un polyglycérol (15 ) dans un rendement quasi quantitatif . Si on a préalablement lavé les résines avant d'évaporer le polyglycérol , la couleur Lovibond est égale à 5. La répartition des polyglycérols est donnée dans le tableau I ci-après .

EXEMPLE 3 :

Essai comparatif de fabrication de pentaglycérol à partir de glycérine bidistillée et d'hydroxyde de sodium .

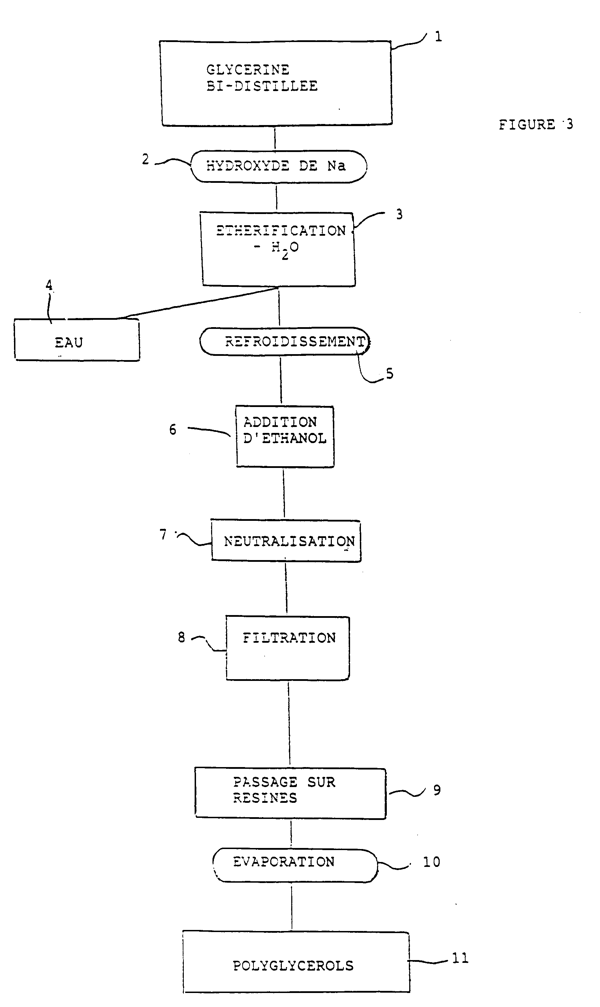

Comme indiqué sur la figure 3 , 460 g de glycérine bidistillée ( 1 ) et 4,6 g d'hydroxyde de sodium ( 2 ) sont chauffés jusqu'à 255°C ( 3 ) . Après 6 h 50 , on recueille 72g d'eau ( 4 ) qui correspond à la stoéchiométrie pour obtenir un pentaglycérol . On refroidit à 150°C ( 5 ) puis après addition de 200 ml d'alcool éthylique ( 6 ) , on neutralise le catalyseur basique par une solution d'acide sulfurique à 50% ( 7 ) (5,635 g d'acide sulfurique à 100% ) . Après filtration ( 8 ) , passage sur résines échangeuses d'ions ( 9 ) , évaporation de l'alcool ( 10 ) , on obtient un polyglycérol (11 ) légèrement trouble à froid ayant une couleur Lovibond égale à 6 . Sa composition est indiquée dans le tableau I (ci-après) .

EXEMPLE 4 :

Fabrication d'un pentaglycérol.

On utilise la même matière première que dans l'exemple 1 , mais on opère à une température de 270°C et en utilisant une colonne à distiller thermostatée à 110°C pour éviter au maximum le reflux de l'eau de réaction.
On constate une diminution sensible de la teneur résiduelle en glycérine ( voir tableau I ci-après ).

EXEMPLE 5 :

Ester mixte de coprah et colza de pentaglycérol .

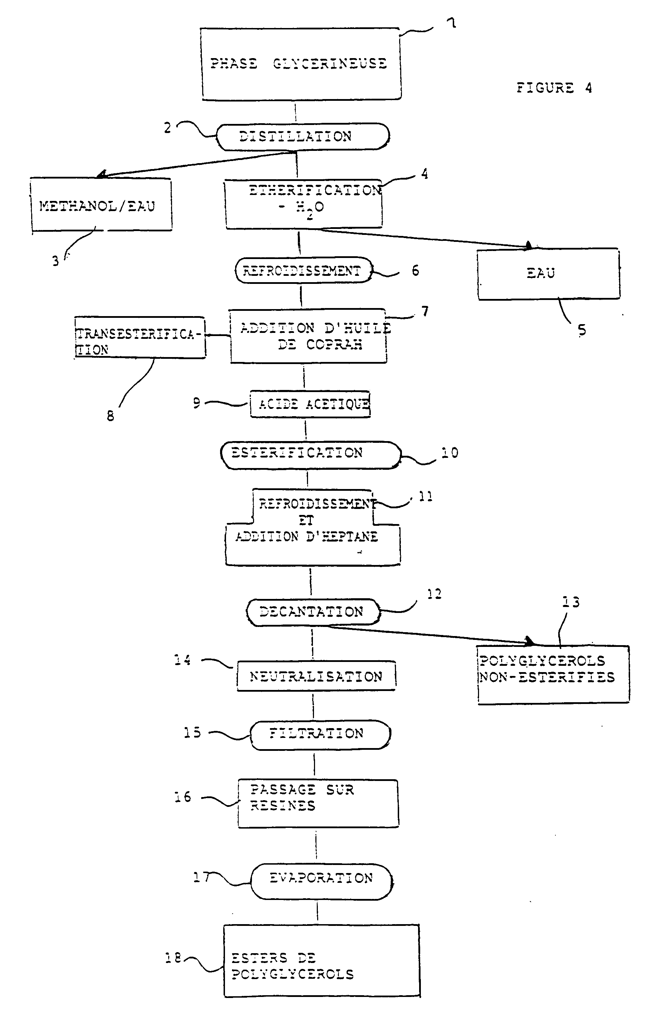

Comme indiqué sur le diagramme de la figure 4 , dans un premier stade on fabrique un polyglycérol selon l'exemple 1. A 200°C, après refroidissement , on injecte de l'huile de coprah puis de l'acide acétique . On refroidit , neutralise et on élimine les sels . Le protocole détaillé complet est le suivant :
- chauffage à 230°C puis 250°C ( 2 ) de la glycérine brute ( 1 ) ( 700 g ) contenant 352,7 g de glycérine ( 3,83 moles ) et 0,37 mole de sel de sodium et récupération de 278,4 g d'un mélange méthanol/eau (3) ,
- évaporation de l'eau de condensation entre 230 et 250°C ( 4 ), récupération de 63,4 g d'eau ( 5 ) puis refroidissement ( 6 ),
- introduction à 200° C d'huile de coprah (120,8 g) (7 ).
- maturation à 200-210°C ( 40') (8) ,
- addition d'acide acétique (22,1 g ) à 215°C ( 9 ),
- estérification des acides gras libérés par l'acide acétique ( 10 ) ,
- refroidissement par ajout d'heptane (11) et en même temps élimination d'eau ,
- décantation ( 12 ) à 80°C des polyglycérols qui n'ont pas réagi (13 ) ,
- neutralisation de la phase supérieure d'ester de polyglycérol + heptane à l'acide sulfurique ( 14 ),
- filtration du sulfate ( 15 ) ,
- passage sur résines ( 16 ) ,
- évaporation de l'heptane ( 17 ) ,
- produit final ( 18 ) : 315 g d'ester de polyglycérol et 89 g de polyglycérol .
Le rendement molaire par rapport à la théorie est de 65% en ester de polyglycérol car tout le polyglycérol n'a pas réagi.

EXEMPLE 6 :

Ester de coprah de polyglycérol.

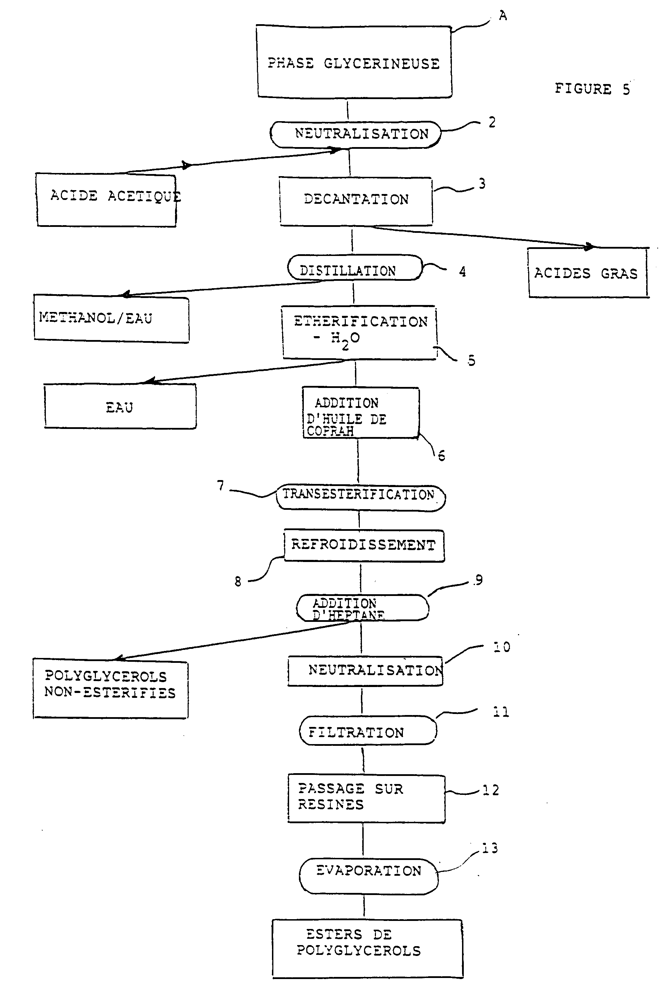

Comme indiqué sur la figure 5 , dans cet exemple on élimine préalablement l'acide gras présent sous forme de sel , ce qui revient à déplacer comme dans l'exemple 2 le sel par l'acide acétique .

5

On ajoute donc 22,05 g d'acide ( 2 ) dans 700 g de phase glycérineuse ( 1 ) et l'on recueille 86,3 g d'un mélange d'acide gras + ester par décantation ( 3 ) .

La suite consiste à :

- évaporer jusqu'à 230°C le méthanol et l'eau libres ( 4 ) ,
- évaporer l'eau de condensation entre 230 et 260°C (56,5 g ) ( 5 ) ,
- à introduire 254,5 g d'huile de coprah en trois fois pour une stoéchiométrie de 1,5 moles de pentaglycérol soit 1,15 moles dans le mélange ( 6 ) ,
- on laisse la réaction se réaliser entre 180 à 220°C ( 1/2 heure) ( 7 ) ,
- on refroidit ( 8 ) et l'on injecte 300 ml d'heptane ( 9 ) puis l'acide sulfurique (10) . On obtient finalement après filtration (11) et passage sur résines (12) et évaporation ( 13 ), 447 g de produit final sous forme d'ester de polyglycérol soit 88% de rendement , tout en recueillant le polyglycérol qui n'a pas réagi ( 60 à 70 g ) .

TABLEAU I

| | T°C | temps | glycérine % | diglycérols % | triglycérol % | tétraglycérol % | pentaglycérols % |
|---|---|---|---|---|---|---|---|
| Exemple 1* | 255 | 3h55 | 28,0 | 27,82 | 20,80 | 12,27 | 5,35 |
| Exemple 2* | 255 | 4h50 | 33,21 | 28,83 | 18,24 | 9,16 | 3,38 |
| Exemple 3 * | 255 | 6h50 | 22,53 | 40,9 | 18,63 | 9,40 | 3,22 |
| Exemple 4 * | 270 | 1h20 | 8,52 | 26,23 | 27,72 | 17,04 | 9,40 |
| A 1 * | 265 | 1h35 | 17,52 | 28,30 | 24,16 | 14,98 | 8,10 |
| A 2 * | 265 | 2h35 | 24,90 | 30,77 | 20,87 | 11,82 | 3,34 |

EP 0 518 765 B1

TABLEAU I (suite)

| | hexaglycérols % | composés cycliques dimères % | couleur Lovibond |
|---|---|---|---|
| Exemple 1 * | 1,50 | 4,08 | 2,5 |
| Exemple 2 * | traces | 4,44 | 5 |
| Exemple 3 * | traces | 4,89 | 6 |
| Exemple 4 * | 2,88 | 8,21 | 5 |
| A 1 * | 1,67 | 5,27 | 3 |
| A 2 * | traces | 6,05 | 5 |

* exemples 1 - 4 phase glycérine brute de transestérification d'huile.
exemple 2 phase glycérine brute de transestérification d'huile neutralisée à l'acide acétique.
exemple 3 glycérine bidistillée + hydroxyde de sodium
A1 idem que l'exemple 1 mais à 265°C.
A2 idem que l'exemple 2 mais à 265°C./

Analyse effectuée sur colonne Cp Sil 5 CB 8m (sur échantillon silylé).
T°C colonne 120 à 220°C à 30°C/min.
puis 220 à 320°C à 20°C/min.

Injection sur colonne - 80 à 300°C à 180°C/min.
Détecteur - 300°C.

## Revendications

1. Procédé pour l'obtention de polyglycérols comprenant l'étape de chauffage d'une matière première glycérineuse brute provenant d'une réaction d'estérification ou de transestérification en soi connue d'une huile et d'un monoalcool en présence de catalyseur basique .

2. Procédé selon la revendication 1 , caractérisé en ce que l'huile est une huile végétale ou animale telle que , par exemple celles de palmiste, coprah , babassu , palme , suif , saindoux , tournesol , colza , lin, arachide , coton , carthame , poisson , soja , tall oil , seules ou en mélanges.

3. Procédé selon l'une des revendications 1 ou 2 , caractérisé en ce que le monoalcool est le méthanol , l'éthanol, le propanol ou le butanol , le méthanol et l'éthanol étant préférés .

4. Procédé selon l'une quelconque des revendications 1 à 3 , caractérisé par le fait que ladite matière première glycérineuse est chauffée jusqu'à 200°C environ puis de 220 à 270°C , pour éliminer d'abord le mélange alcool libre - eau puis l'eau de condensation parvenant de la formation de polyglycérols .

5. Procédé selon l'une quelconque des revendications 1 à 3, caractérisé par le fait qu'on traite ladite matière première glycérineuse par un acide carboxylique faible dont le pK est inférieur à celui d'un acide gras , tel que l'acide acétique , on effectue une décantation pour séparer acide gras et composés huileux après ou avant évaporation de l'alcool présent dans ladite matière , puis on chauffe jusqu'à 200°C environ pour éliminer l'eau et l'alcool libres encore présents et enfin entre 220 et 270°C pour éliminer l'eau de condensation des polyglycérols , qui se forment dans cette gamme de température .

6. Procédé selon l'une quelconque des revendications 1 à 5, caractérisé par le fait qu'on élimine le catalyseur avec un acide fort tel que l'acide sulfurique par formation d'un sel insoluble dans un alcool à basse température, filtration, décantation et passage sur résines en présence d'eau .

7. Procédé selon l'une quelconque des revendications 1 à 6 , caractérisé par le fait que le polyglycérol obtenu est traité avec un acide gras , un ester gras ou une huile , entre 150 et 250°C , ce qui conduit à un ester de polyglycérol.

8. Procédé selon la revendication 7 , caractérisé par le fait que , avant l'addition d'un composé huileux sur le polyglycérol , on traite celui-ci par un acide carboxylique de type acide faible entre 120 et 150°C et décantation des acides gras .

9. Procédé selon la revendication 7 , caractérisé par le fait que l'on ajoute entre 180 et 220°C , avant ou après addition du composé huileux , un acide carboxylique, tel que l'acide acétique , pour déplacer le sel d'acide gras et faire réagir l'acide gras provenant du sel .

10. Procédé selon l'une quelconque des revendications 1 à 9 , caractérisé par le fait qu'on élimine les ions sodium ou potassium , par addition d'acide fort en milieu hydrocarboné, filtration du sel formé , décantation des polyglycérols qui n'ont pas réagi et purification éventuelle de la phase ester de polyglycérol.

11. Procédé selon l'une quelconque des revendications 1 à 10 , caractérisé par le fait que l'on distille , après la formation de polyglycérol , la glycérine et les composés cycliques de bas poids moléculaire , en soumettant le polyglycérol à une distillation sous vide pour entraîner glycérine et composés cycliques.

**Claims**

1. Process for the preparation of polyglycerols and polyglycerol esters comprising the heating phase of a raw glycerinous material resulting from a, well-known in itself, esterification or transesterification reaction of an oil or a monohydric alcohol exposed to a basic catalyst.

2. Process according to claim 1, characterized in that the oil is a vegetable or animal oil such as for instance, extracted from palm-tree, copra, babasu, palm, tallow, lard, sunflower, rapeseed, linseed, peanut, cotton seed, safflower, fish, soya bean, on their own or in mixtures.

3. Process according to one of the claims 1 or 2, characterized in that the monohydric alcohol is methanol, ethanol, propanol or butanol, whereas methanol and ethanol should be used preferably.

4. Process according to any one of the claims 1 to 3, characterised in that said raw glycerinous material is heated to approx. 200°C, then between 220 and 270°C, in order first of all to eliminate the free alcohol/water mixture, then condensation water resulting from the formation of polyglycerols.

5. Process according to any one of the claims 1 to 3, characterized in that said glycerinous material is treated with a weak carboxylic acid, whose pK is less than that of a fat acid, such as acetic acid, whereby decantation is carried out in order to separate fat acid from oily compounds, after or before evaporation of the alcohol present in said material, then the mixture is heated to approx. 200° in order to eliminate free alcohol and water still present, and then between 220 and 270°C in order to eliminate the condensation water resulting from the polyglycerols, which usually build up over this temperature range.

6. Process according to any one of the claims 1 to 5, characterised in that the catalyst is eliminated by applying a strong acid, such as sulphuric acid, by formation of a soluble salt in alcohol at low temperature, filtering, decantation and passing over resins in the presence of water.

7. Process according to any one of the claims 1 to 6, characterised in that the polyglycerol thus prepared is treated with a fat acid, a fat ester or an oil, between 150 and 250°C, which produces a polyglycerol ester.

8. Process according to claim 7, characterized in that, before adding an oily compound onto the polyglycerol, it is treated with a carboxylic acid, with low acidity, between 120 and 150°C, and decantation of the fat acids.

9. Process according to claim 7, characterized in that one adds, between 180 and 220°C, before or after the addition of the oily compound, a carboxylic acid, such as acetic acid, in order to displace the fat acid salt and cause the reaction of the fat acid from the salt.

10. Process according to any one of the claims 1 to 9, characterised in that one eliminates the sodium or potassium ions, by adding a strong acid in a hydrocarboned medium, filtering of the salt thus formed, decantation of the polyglycerols which have not reacted and, if needed, purification of the polyglycerol ester phase.

11. Process according to any one of the claims 1 to 10, characterised in that, after formation of polyglycerol, the glycerin and the cyclic compounds with low molecular weight are distillated, by exposing the polyglycerol to vacuum distillation in order to drive the glycerin as well as the cyclic compounds.

**Patentansprüche**

1. Verfahren zur Gewinnung von Polyglycerinen, umfassend die Stufe des Erhitzens eines Rohglycerin-Rohstoffs, der aus einer an sich bekannten Veresterungs- oder Umesterungsreaktion eines Öls und eines Monoalkohols in Gegenwart eines basischen Katalysators stammt.

2. Verfahren gemäß Anspruch 1, dadurch gekennzeichnet, daß das Öl ein pflanzliches oder tierisches Öl, wie zum Beispiel Palmkernöl, Kopraöl, Babassuöl, Palmöl, Talg, Schweineschmalz, Sonnenblumenöl, Rapsöl, Leinöl, Erdnußöl, Baumwollöl, Färberdistelöl, Fischöl, Sojaöl, Tallöl, einzeln oder in Gemischen, ist.

3. Verfahren gemäß einem der Ansprüche 1 oder 2, dadurch gekennzeichnet, daß der Monoalkohol Methanol, Ethanol, Propanol oder Butanol ist, wobei Methanol und Ethanol bevorzugt werden.

4. Verfahren gemäß einem der Ansprüche 1 bis 3, dadurch gekennzeichnet, daß der Glycerin-Rohstoff auf ungefähr 200°C, dann auf 220 bis 270°C erhitzt wird, um zunächst das Gemisch freier Alkohol/Wasser und dann das Kondensationswasser, das von der Bildung von Polyglycerinen herstammt, zu entfernen.

**5.** Verfahren gemäß einem der Ansprüche 1 bis 3, dadurch gekennzeichnet, daß man den Glycerin-Rohstoff mit einer schwachen Carbonsäure, wie Essigsäure, deren pK kleiner als der einer Fettsäure ist, behandelt, vor oder nach der Verdampfung des im Rohstoff vorhandenen Alkohols dekantiert, um Fettsäure und ölige Verbindungen voneinander zu trennen, dann auf ungefähr 200°C erhitzt, um freies Wasser und freien Alkohol, die noch vorhanden sind, zu entfernen, und schließlich auf zwischen 220 und 270°C erhitzt, um das Wasser aus der Kondensation der Polyglycerine, die sich in diesem Temperaturbereich bilden, zu entfernen.

**6.** Verfahren gemäß einem der Ansprüche 1 bis 5, dadurch gekennzeichnet, daß man den Katalysator mit einer starken Säure, wie Schwefelsäure, durch Bildung eines Salzes, das bei niedriger Temperatur in einem Alkohol unlöslich ist, Filtration, Dekantieren und Leiten über Harze in Gegenwart von Wasser eliminiert.

**7.** Verfahren gemäß einem der Ansprüche 1 bis 6, dadurch gekennzeichnet, daß das erhaltene Polyglycerin zwischen 150 und 250°C mit einer Fettsäure, einem Fettsäureester oder einem Öl behandelt wird, was zu einem Polyglycerinester führt.

**8.** Verfahren gemäß Anspruch 7, dadurch gekennzeichnet, daß man vor dem Hinzufügen einer öligen Verbindung zum Polyglycerin dieses zwischen 120 und 150°C mit einer Carbonsäure des Typs schwache Säure behandelt und die Fettsäuren dekantiert.

**9.** Verfahren gemäß Anspruch 7, dadurch gekennzeichnet, daß man zwischen 180 und 220°C vor oder nach der Zugabe der öligen Verbindung eine Carbonsäure, wie Essigsäure, hinzufügt, um die Fettsäure aus dem Salz zu verdrängen und die aus dem Salz stammende Fettsäure zur Reaktion zu bringen.

**10.** Verfahren gemäß einem der Ansprüche 1 bis 9, dadurch gekennzeichnet, daß man Natrium- und Kalium-Ionen durch Zugabe von starker Säure in Kohlenwasserstoffmedium, Abfiltrieren des gebildeten Salzes, Dekantieren der Polyglycerine, die nicht reagiert haben, und gegebenenfalls Reinigung der Polyglycerinesterphase entfernt.

**11.** Verfahren gemäß einem der Ansprüche 1 bis 10, dadurch gekennzeichnet, daß man nach der Bildung von Polyglycerin Glycerin und cyclische Verbindungen mit niedrigem Molekulargewicht abdestilliert, indem man das Polyglycerin einer Vakuumdestillation unterwirft, um Glycerin und cyclische Verbindungen auszutreiben.

FIGURE 1

FIGURE 2

PHASE GLYCERINEUSE — 1

ACIDE ACETIQUE — 2

DECANTATION — 3

ACIDES GRAS
4

DISTILLATION — 5

METHANOL/EAU — 6

ETHERIFICATION
- $H_2O$
7

EAU
8

REFROIDISSEMENT — 9

ADDITION
D'ETHANOL — 10

NEUTRALISATION — 11

FILTRATION — 12

PASSAGE SUR
RESINES — 13

EVAPORATION — 14

POLYGLYCEROLS — 15

FIGURE 3

FIGURE 4

FIGURE 5

PHASE GLYCERINEUSE — A

NEUTRALISATION — 2

ACIDE ACETIQUE

DECANTATION — 3

ACIDES GRAS

DISTILLATION — 4

METHANOL/EAU

ETHERIFICATION - $H_2O$ — 5

EAU

ADDITION D'HUILE DE COPRAH — 6

7 — TRANSESTERIFICATION

REFROIDISSEMENT

8

9 — ADDITION D'HEPTANE

POLYGLYCEROLS NON-ESTERIFIES

NEUTRALISATION — 10

FILTRATION — 11

PASSAGE SUR RESINES — 12

EVAPORATION — 13

ESTERS DE POLYGLYCEROLS